# EUROPÄISCHE PATENTANMELDUNG

(11) **EP 3 772 352 A1**
(43) Veröffentlichungstag der Anmeldung: **10.02.2021**
(21) Anmeldenummer: 19190022.4
(22) Anmeldetag: 05.08.2019
(51) Int. Cl.: A61F 2/915, A61F 2/24

(54) **IMPLANTAT MIT INTEGRIERTER GELENKSTRUKTUR**

(71) Anmelder: BIOTRONIK AG, 8180 Bülach (CH)
(72) Erfinder: Rothstock, Stephan, 13353 Berlin (DE); Koop, Karsten, 18057 Rostock (DE); Hein, André, 18055 Rostock (DE); Goebel, Paul, 18059 Rostock (DE); Flores, Imanol, 18059 Rostock (DE); Hof, Andreas, 23568 Luebeck (DE)
(74) Vertreter: Galander, Marcus

(57) **Zusammenfassung**

Die Erfindung betrifft ein Implantat (10), beispielsweise eine Herzklappenprothese, mit einer durchbrochenen, hohlzylinderförmigen und/oder hohlkegelstumpfförmigen Grundstruktur (20), die sich aus einer Vielzahl von Streben (21) und die Streben (21) miteinander verbindenden Knoten (24) zusammensetzt, wobei an jedem Knoten (24) jeweils mindestens zwei Streben (21) miteinander verbunden sind. Um eine sichere Abdichtung des Gefäßes oder Annulus, in das das Implantat eingebracht ist, zu gewährleisten, weist mindestens ein Teil der Knoten (24) jeweils eine kreuzförmige durchgehende Aussparung (27) auf, welche die Biegeflexibilität der Grundstruktur (20) erhöht.

## Beschreibung

Die vorliegende Erfindung betrifft ein Implantat mit einer durchbrochenen, hohlzylinderförmigen und/oder hohlkegelstumpfförmigen Grundstruktur, die sich aus einer Vielzahl von Streben und einer Vielzahl von die Streben miteinander verbindenden Knoten (Verbinder) zusammensetzt, wobei an jedem Knoten jeweils mindestens zwei Streben miteinander verbunden sind. Das Implantat nimmt einen komprimierten Zustand und einen expandierten Zustand ein.

Medizinische Implantate (Prothesen), insbesondere intraluminale Endoprothesen, für unterschiedlichste Anwendungen sind in großer Vielfalt aus dem Stand der Technik bekannt. Prothesen sind z.B. Endoprothesen zum Verschließen von persistierenden Foramen Ovale (PFO), Endoprothesen zum Verschließen eines ASG (Vorhofscheidewanddefekt, Atrioseptaldefekt), Aortenprothesen sowie Prothesen im Bereich des Hart- und Weichgewebes. Ein prominenter Vertreter der Prothesen sind Herzklappenprothesen, welche als künstlicher Ersatz für eine natürliche Herzklappe zur Verhinderung einer akuten oder chronischen Herzinsuffizienz verwendet werden. Herzklappenprothesen werden nach der offen-chirurgischen Technik oder in letzter Zeit zunehmend minimal-invasiv eingesetzt.

Für die Anwendung minimal-invasiver Verfahren im Bereich des Herzklappenersatzes ist heute insbesondere die Transkatheter-Aortenklappenimplantation (englisch: transcatheter aortic valve implantation TAVI) von Bedeutung. Bei dieser Implantationstechnik wird mittels eines Katheters eine Herzklappenprothese für die Aortenklappe u.a. transfemoral oder transkapital eingesetzt. Eine solche Herzklappenprothese besitzt häufig eine durchbrochene hohlzylinder- und/oder hohlkegelstumpfförmige (röhrenförmige) Grundstruktur als Stützstruktur, innerhalb der Grundstruktur angebrachte biegsame Klappensegel (Leaflet) sowie einen Skirt, welches den Bereich des Annulus an der natürlichen Herzklappe nach dem Einsetzen in das Herz abdichtet, um ein Rückfließen des Bluts nach Ausstoß in das Gefäß (Aorta) zu vermeiden. Hierfür ist das Skirt an der Grundstruktur befestigt und verschließt den Zwischenraum zwischen Annulus und der Grundstruktur. Die Grundstruktur selbst ist an beiden Enden in Längsrichtung offen.

Als Implantate werden außerdem häufig Stents eingesetzt, die zur Behandlung von Stenosen (Gefäßverengungen) dienen. Sie weisen meist eine durchbrochene hohlzylinderförmige (rohrförmige) Grundstruktur auf, die an beiden Enden in Längsrichtung offen ist. Ein derartiges Implantat wird häufig mittels eines Katheters in das zu behandelnde Gefäß eingesetzt und dient dazu, das Gefäß über einen längeren Zeitraum (Monate bis Jahre) zu stützen. Durch den Einsatz von Stents können verengte Bereiche in den Gefäßen erweitert werden.

Die Grundstruktur von oben beschriebenen Implantaten setzt sich häufig aus Zellen (Maschen) zusammen, welche aus einer Vielzahl von Streben (Stege, Struts), beispielsweise aus zick-zack- oder mäanderförmige Strukturen formenden Streben, gebildet werden. An den in Längsrichtung bzw. den Seiten angeordneten Enden der Zellen treffen sich jeweils zwei oder mehr als zwei Streben in einem sogenannten Knoten, welche die Zellen und die Streben miteinander verbinden.

Ein derartiges Implantat, welches kathetergestützt eingesetzt wird, beziehungsweise seine Grundstruktur nimmt üblicherweise zwei Zustände ein, nämlich einen komprimierten Zustand mit einem kleinen Durchmesser und einen expandierten Zustand mit einem größeren Durchmesser. Im komprimierten Zustand kann das Implantat mittels des Katheters durch enge Gefäße hindurch in das Herz oder das Gefäß eingeführt und z.B. im Bereich der Aortenklappe oder der Venenklappe positioniert werden. Der Übergang von dem komprimierten Zustand in den expandierten Zustand erfolgt durch Expandieren der Stützstruktur beispielsweise mittels eines Ballons. Die Expansion der Stützstruktur bewirkt bei der Herzklappenprothese deren Entfaltung. Alternativ erfolgt der Übergang in den expandierten Zustand bei einem selbstexpandierenden Implantat bei einer Überschreitung der Transformationstemperatur.

Mittels des Katheters wird die Herzklappenprothese in die richtige Position gebracht und beispielsweise nach Zurückziehen einer Katheterhülse in proximale Richtung entfaltet. Hierbei muss die Katheterhülse jedoch in manchen Situationen auch zumindest teilweise wieder in distale Richtung über die Herzklappenprothese geschoben werden (Resheathing, Wiedereinfangen), um eine Repositionierung der Herzklappenprothese zu ermöglichen.

Die Dichtigkeit eines Implantats kann bei Verkalkungen und Ablagerungen an dem jeweiligen Gefäßes oder des Herzens, das heißt z.B. bei Abweichungen des Annulus von der Kreisform, je nach Design des Implantats, stark variieren. Dafür ist eine hohe Biegeflexibilität des Implantats vorteilhaft, so dass sich das Implantat verschiedenen Anatomien anpassen kann. Ferner werden die Implantate an der AuOßenseite mit einem Skirt versehen, der geeignet ist, sich den verschiedenen Anatomien anzupassen. Stents mit zu wenig Radialkraft oder sehr großen Zellstrukturen, die sich der Annulusform bzw. Verkalkungen nicht anpassen, neigen zu paravalvularen Leckagen, die für den Patienten eine lebensbedrohliche Situation hervorrufen können. Derzeit erscheint es nicht realisierbar, die Form des Implantats vor dem Einsetzen exakt der individuellen Form des Gefäßes bzw. des Annulus anzupassen.

Zudem besteht das Problem, dass herkömmliche Implantate beim Resheathen starke axiale und radiale Kräfte erzeugen, welche zu bleibenden Verformungen am Katheter führen können. Damit ist die Ausbildung von hohen Radialkräften durch ein Implantat nicht erwünscht, weil die verwendeten Kathetersysteme hohen Resheathingkräften nur begrenzt standhalten können.

Die Aufgabe der vorliegenden Erfindung besteht somit darin, ein Implantat zu schaffen, welches die obigen Nachteile vermeidet und insbesondere eine sichere Abdichtung des Gefäßes oder Annulus, in das das Implantat eingebracht ist, gewährleistet.

Die obige Aufgabe wird gelöst durch das Implantat mit den Merkmalen des Anspruchs 1.

Das erfindungsgemäße Implantat besitzt insbesondere eine Grundstruktur, bei der mindestens ein Teil der Knoten jeweils eine kreuzförmige durchgehende Aussparung aufweist. Die kreuzförmige Aussparung ist derart in dem Knoten angebracht, dass sie vollständig von Material der Grundstruktur umgeben oder umschlossen ist. Die Aussparung geht in radialer Richtung der Grundstruktur durch das Material der Grundstruktur im Bereich eines Knotens hindurch. Das erfindungsgemäße Implantat ist vorzugsweise eine Herzklappenprothese oder eine Venenklappenprothese. Bei einer Ausbildung des Implantats als Herzklappenprothese umfassend eine Grundstruktur bzw. ein Grundgerüst (Stent) und eine darin befestigte Klappenanordnung kommen die Vorteile der vorliegenden Erfindung besonders zum Tragen.

Die erfindungsgemäßen Aussparungen verringern das Flächenträgheitsmoment der Knoten (Verbinder) und erhöhen somit die Biegeflexibilität verglichen mit herkömmlichen Knoten ohne eine derartige Aussparung. Die Grundstruktur ist somit biegeweicher und passt sich besser unregelmäßigen verkalkten Anatomien an. Im Vergleich zu anderen Grundstrukturen ergibt sich, dass der erfindungsgemäße Knoten mit kreuzförmiger Aussparung eine relativ hohe Stabilität bei Zug- oder Druckkräften aufweist, die auf die Grundstruktur des Implantats wirken, wie sie im Katheter oder bei Belastung mit den Herzklappenkräften auftreten können. Auch die Torsionsstabilität der Knoten ist gewährleistet. Zudem besitzt die Grundstruktur des erfindungsgemäßen Implantats, wie unten noch genauer erläutert wird, geringere Resheathingkräfte, die zudem in vorteilhafter Weise lediglich allmählich (d.h. nicht sprunghaft) ansteigen.

Die Grundstruktur besteht vorzugsweise aus einer Formgedächtnislegierung, beispielsweise Nitinol.

In einem bevorzugten Ausführungsbeispiel sind an einem Knoten mindestens zwei Streben, weiter bevorzugt drei und weiter vorzugsweise mindestens vier Streben miteinander verbunden. Bei der Grundstruktur bilden die Streben Maschen oder Zellen aus, beispielsweise vier Streben eine rautenförmige Zelle. Die Knoten sind jeweils an den Enden der Maschen oder Zellen angeordnet.

Eine Weiterbildung der Erfindung zeichnet sich dadurch aus, dass jede kreuzförmige Aussparung einen mittig angeordneten Zentralabschnitt sowie mindestens vier von diesem Zentralabschnitt kreuzförmig abgehende Seitenabschnitte (Arme) aufweist, die insgesamt das "Kreuz" ausbilden. Mit einem anderen Wort kann die Aussparung als im weiteren Sinne schmetterlingsförmig beschrieben werden. Die Breite b des Zentralabschnitts in Umfangsrichtung beträgt mindestens 0,2, vorzugsweise 1,5 und maximal 5 Mal die Ausdehnung der Länge l des Zentralabschnitts. In einem weiteren Ausführungsbeispiel der Erfindung beträgt die Länge jedes Zentralabschnitts dieselbe Ausdehnung wie die Breite des Zentralabschnitts in Umfangsrichtung.

In einem weiteren Ausführungsbeispiel ist jeder Seitenabschnitt an dem Zentralabschnitt gegenüber liegenden Ende kreisabschnittsförmig ausgebildet. In einem weiteren Ausführungsbeispiel ist jeder Seitenabschnitt gleich lang.

In einer Weiterbildung der Erfindung beträgt die Breite A des Knotens in Längsrichtung 0,2 bis 5 Mal die Breite B des Knotens in Umfangsrichtung U. Hierdurch wird eine vergleichsweise hohe Stabilität der Grundstruktur in Bezug auf Zug- oder Druckkräfte, wie sie im Katheter oder bei Belastung nach Implantation auftreten können, gewährleistet. In einer Ausführungsform beträgt die Breite A des Knotens in Längsrichtung die Breite B des Knotens in Umfangsrichtung U.

In einem weiteren Ausführungsbeispiel ragen in die Aussparung zwei einander gegenüber liegende Vorsprünge hinein, die sich in Umfangsrichtung erstrecken. Alternativ oder zusätzlich ragen in die Aussparung zwei einander gegenüber liegende Vorsprünge hinein, die sich in Längsrichtung erstrecken. Diese Vorsprünge begrenzen durch ihren Abstand insbesondere die Biegeflexibilität der Grundstruktur in die jeweilige Richtung.

Es ist weiter von Vorteil, dass die kreuzförmige durchgehende Aussparung in jedem Knoten eines Abschnitts der Grundstruktur vorgesehen ist, der im bestimmungsgemäßen Gebrauch des Implantates (nach Implantation) den Einströmbereich des Implantates bildet. Im der bevorzugten Ausgestaltung der Erfindung als Herzklappenprothese wird bei Implantation die Prothese an die Stelle einer natürlichen Herzklappe beispielsweise der Aortenklappe implantiert. Die Grundstruktur der Herzklappenprothese sitzt dann im Aortenannulus und die darin angeordnete Klappenanordnung übernimmt die Funktion der natürlichen Aortenklappe. Das Blut strömt vom linken Ventrikel in der Offenstellung der Klappe durch die Grundstruktur in die Aorta. Der Rückstrom von der Aorta wird durch die Klappenanordnung blockiert. Der Bereich der Grundstruktur der in Richtung des vom Ventrikel strömenden Blutes von der Klappenanordnung liegt, wird als Einströmbereich des Implantates (in diesem Fall der Herzklappenprothese) bezeichnet.

In einem Ausführungsbeispiel der Erfindung sind an der Grundstruktur mindestens zwei Klappensegel und/oder ein Skirt befestigt. Die Befestigung erfolgt dabei vorzugsmäßig durch Nähen oder Kleben.

Weitere Ziele, Merkmale, Vorteile und Anwendungsmöglichkeiten der Erfindung ergeben sich auch aus der nachfolgenden Beschreibung von Ausführungsbeispielen der Erfindung anhand der Figuren. Dabei bilden alle beschriebenen und/oder bildlich dargestellten Merkmale für sich oder in beliebiger Kombination den Gegenstand der Erfindung, auch unabhängig von ihrer Zusammenfassung in den einzelnen Ansprüchen oder deren Rückbezug.

Es zeigen schematisch
- Fig. 1: ein erfindungsgemäßes Implantat in einer perspektivischen Ansicht von der Seite;
- Fig. 2: das Implantat gemäß Fig. 1 nach der Implantation im Bereich einer natürlichen Aortenklappe in einer teilweise geschnittenen Darstellung;
- Fig. 3: die Grundstruktur eines erfindungsgemäßen Implantats in einer perspektivischen Ansicht von der Seite;
- Fig. 4 - 5: einen Ausschnitt der Grundstruktur gemäß Fig. 3 im Bereich eines Knotens; und
- Fig. 6: ein Diagramm, in dem der Radialkraftverlauf (in N) über dem Durchmesser (in mm) einer Grundstruktur ohne spezielle Knotenformen im Vergleich mit einer erfindungsgemäßen Grundstruktur beim Crimpen vom Nenndurchmesser auf 18 French und Freilassen auf den Nenndurchmesser eingezeichnet ist.

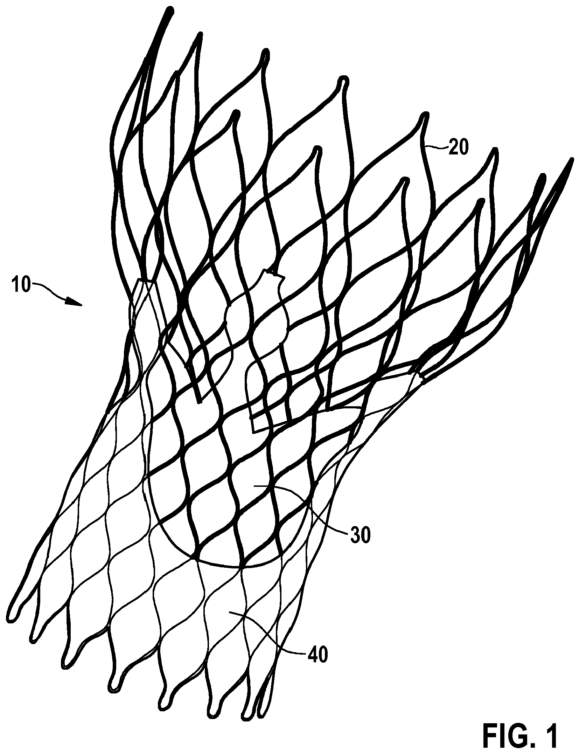

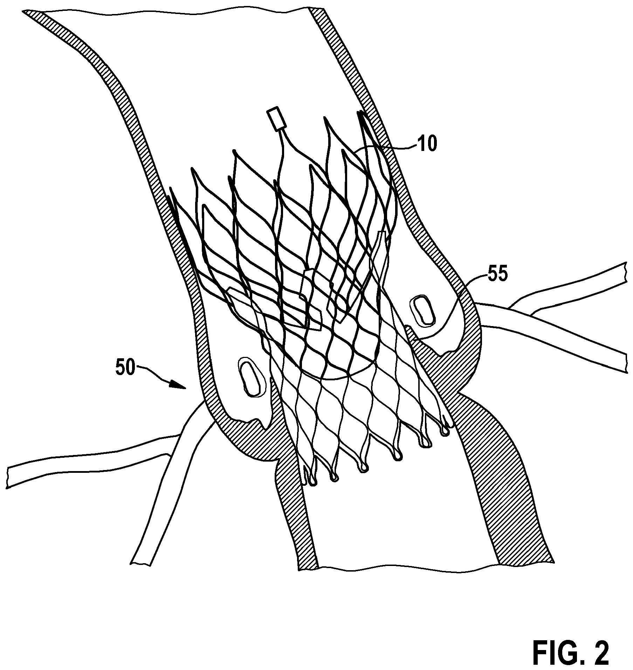

In Fig. 1 ist ein erfindungsgemäßes Implantat in Form einer Herzklappenprothese 10 mit einer Grundstruktur 20 dargestellt. Die Grundstruktur 20 (auch als Herzklappenstent bezeichnet) bildet ein stützendes Gerüst für das Implantat. Die Grundstruktur 20 ist ein hohlzylinderförmiges Gerüst, das entlang der Längsrichtung L in seinem Durchmesser variiert kann, um, wie Fig. 2 zeigt, optimal in die durch den Patienten anatomisch vorgegebene Struktur eingepasst zu werden. Alternativ oder zusätzlich kann die Grundstruktur 20 hierfür mindestens einen hohlkegelstumpfförmigen Abschnitt aufweisen.

Die Herzklappenprothese 10 (Fig. 1) besteht beispielsweise aus einer Formgedächnislegierung, hat einen kleineren Durchmesser der im Annulusbereich positioniert wird und einen größeren Durchmesser der in die aufsteigende Aorta reicht. Beide Bereiche werden durch eine s-förmige Übergangszone verbunden. Die Herzklappenprothese 10 weist ferner drei Klappensegel 30 sowie ein Skirt 40 auf, welche mittels Vernähen an der Grundstruktur 20 befestigt sind. Das Skirt 40 soll die Herzklappenprothese 10 so abdichten, dass das Blut, dass die Herzklappenprothese 10 passiert hat, nicht seitlich an der Herzklappenprothese 10 vorbeifließen kann.

Fig. 2 zeigt die Anordnung einer erfindungsgemäßen Herzklappenprothese 10 nach dem Implantieren im Bereich des Annulus 50 einer Aortenklappe. Die natürliche Herzklappe 55 wird hierbei durch die künstliche Herzklappenprothese 10 verdrängt. Die Herzklappenprothese 10 übernimmt die Funktion der natürlichen Herzklappe 55 sobald die Implantation abgeschlossen ist.

Da im Bereich des Annulus 50 eine unregelmäßige Anatomie beispielsweise aufgrund von vorhandenen Verkalkungen vorliegen kann, ist es für eine ordnungsgemäße Funktion der Herzklappenprothese 10 erforderlich, dass die Grundstruktur 20 im Bereich des Inflows eine hohe Biegeflexibilität aufweist, um sich an eine derartige Geometrie anpassen zu können. Hierdurch werden paravalvulare Leckagen vermieden.

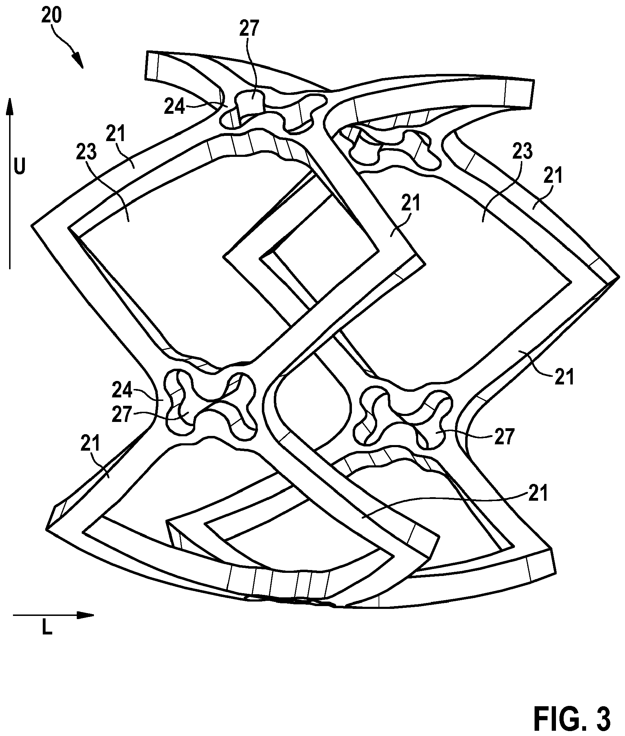

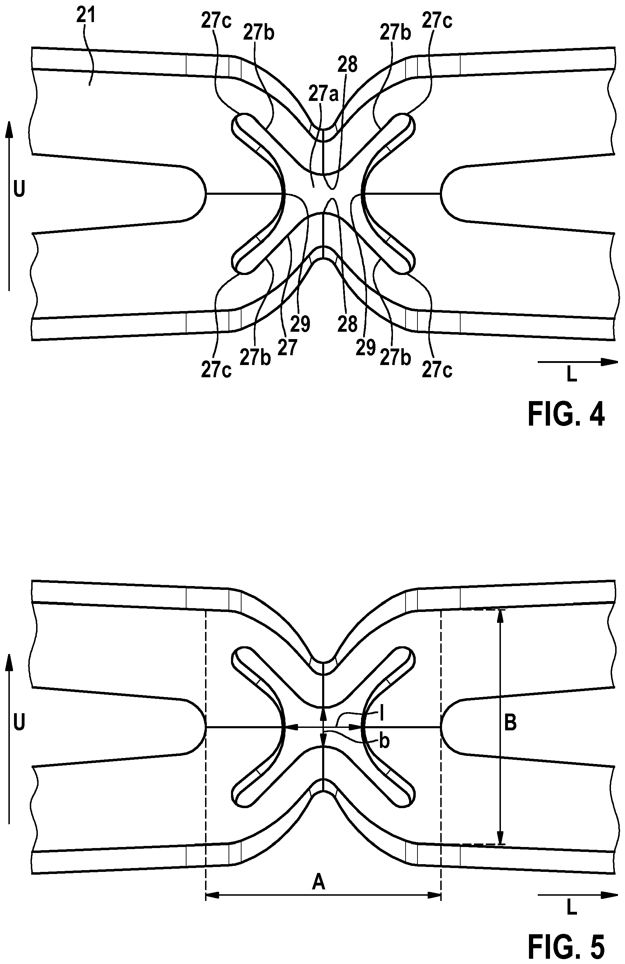

Um eine derartige hohe Biegeflexibilität der Grundstruktur 20 zumindest im Bereich des Inflows zu gewährleisten, weist die Grundstruktur 20 zumindest in diesem Bereich jeweils eine kreuzförmige oder schmetterlingsförmige durchgehende Aussparung (Öffnung) 27 auf. Eine derartige Aussparung ist in den Figuren 3 und 4 im Detail gezeigt, in denen Ausschnitte des Implantats mit Grundstruktur 20 gezeigt sind, die aus einer Vielzahl von Streben 21 besteht, welche über Knoten 24 miteinander verbunden sind. Jeweils vier Streben 21 bilden, wie Fig. 4 zu entnehmen ist, rautenförmige Zellen oder Maschen 23 aus. Jede Aussparung 27 besitzt einen Zentralabschnitt 27a, der etwa mittig in der kreuzförmigen Aussparung 27 angeordnet ist und den Schnittpunkt der vier Seitenabschnitte 27b der Aussparung 27 bildet. Jeder Seitenabschnitt 27b bildet ein Ende 27c aus, das dem Zentralabschnitt 27a gegenüberliegt. Der Zentralabschnitt 27a wird in Umfangsrichtung U begrenzt durch zwei einander gegenüberliegende Vorsprünge 28, deren Abstand als Breite b (siehe Fig. 5, die der besseren Übersicht halber gesondert zu Fig. 4 angeführt wird. Hier werden die Ausmaße A und B des Knotens, b und l des Zentralabschnittes ersichtlich) des Zentralabschnitts 27a bezeichnet wird. In Längsrichtung L wird der Zentralabschnitt 27a der Aussparung 27 durch die einander gegenüberliegenden Vorsprünge 29 begrenzt, deren Abstand als Länge l des Zentralabschnitts 27a bezeichnet wird.

Wie in Fig. 3 gezeigt ist, kann die Form der Aussparung 27 derart gestaltet sein, dass die Aussparung 27 an den Enden 27c jedes Seitenabschnitts 27b kreisabschnittsförmig gestaltet ist.

Die erfindungsgemäßen Aussparungen 27 verringern das Flächenträgheitsmoment des jeweiligen Knotens 24 und erhöhen somit die Biegeflexibilität verglichen mit Knoten ohne eine solche Aussparung. Die Grundstruktur 20 ist somit biegeweicher und passt sich besser einer unregelmäßigen Anatomie an. Auch die Torsionsstabilität der Knoten 24 ist gewährleistet.

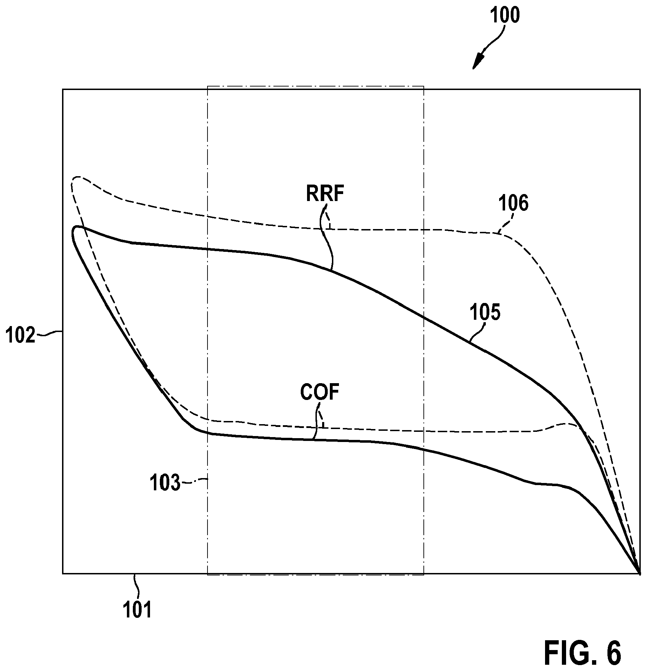

Außerdem haben die Aussparungen einen Effekt auf die Radialkraftkurve (Hysterese), wie dies anschaulich aus dem in Fig. 6 gezeigten Diagramm hervorgeht. In dem Diagramm werden eine Radialkraftkurve 105 eines erfindungsgemäßen Implantats und eine Radialkraftkurve 106 eines Implantats, das in den Knoten eine kreuzförmige Aussparung nicht aufweist, miteinander verglichen. Hierbei ist auf der X-Achse 101 der Durchmesser der Grundstruktur (in mm) und auf der Y-Achse 102 die Radialkraft (in N) aufgetragen. Der mit einer Strich-Punkt-Linie umrandeten Bereich 103 bezeichnet den Arbeitsbereich des erfindungsgemäßen Implantats klinisch in vivo.

Aus dem Diagramm 100 ergibt sich, dass bei gleichem COF (Chronic Outward Force), welcher für den Anpressdruck des Stents in der Aorta sorgt, das erfindungsgemäße Implantat ein geringeres RRF (Radial Resistive Force) aufweist, welcher für die Reaktionskräfte beim Crimpen und Resheathen verantwortlich ist. Damit wird die Hysterese schmaler und es muss weniger Verformungsarbeit beim Freilassen und Resheathen verrichtet werden, was kleinere Resheathing-Kräfte bedingt. Außerdem verläuft der Anstieg des RRF langsamer, was dazu führt, dass die erforderliche Resheathing-Kraft nicht sprunghaft sondern allmählich ansteigt. Es befindet sich demnach immer nur ein kleiner Teil des Implantats mit geringem Durchmesser auf dem oberen RRF-Plateau während der andere Teil des Implantats mit einem größeren Durchmesser im Bereich des unteren RRF-Plateau verbleibt. Eine Limitation des Prinzips besteht darin, dass auch das COF bei größeren Durchmessern leicht abfällt. Dies kann durch größere Durchmesser der Grundstruktur kompensiert werden, so dass der klinische Einsatzbereich wieder im konstanten COF Bereich liegt.

### Bezugszeichenliste

- 10: Herzklappenprothese
- 20: Grundstruktur
- 21: Strebe
- 23: Zelle
- 24: Knoten
- 27: Aussparung
- 27a: Zentralabschnitt der Aussparung 27
- 27b: Seitenabschnitt der Aussparung 27
- 27c: Ende des Seitenabschnitts 27b der Aussparung 27
- 28: Vorsprung
- 29: Vorsprung
- 30: Klappensegel
- 40: Skirt
- 50: Annulus
- 55: natürliche Herzklappe
- 100: Diagramm
- 101: x-Achse
- 102: y-Achse
- 103: Arbeitsbereich des Implantats
- 105: Radialkraftkurve des erfindungsgemäßen Implantats
- 106: Radialkraftkurve eines Vergleichsimplantats ohne Aussparung

In Anbetracht der gesamten obigen Offenbarung, umfasst die vorliegende Erfindung ferner die folgenden nummerierten Ausführungsformen:
1. Implantat (10) mit einer durchbrochenen, hohlzylinderförmigen und/oder hohlkegelstumpfförmigen Grundstruktur (20), die sich aus einer Vielzahl von Streben (21) und die Streben (21) miteinander verbindenden Knoten (24) zusammensetzt, wobei an jedem Knoten (24) jeweils mindestens zwei Streben (21) miteinander verbunden sind, dadurch gekennzeichnet, dass
   mindestens ein Teil der Knoten (24) jeweils eine kreuzförmige durchgehende Aussparung (27) aufweist.
2. Implantat nach Ausführungsform 1, dadurch gekennzeichnet, dass an einem Knoten (24) mindestens zwei Streben (21), vorzugsweise mindestens vier Streben (21) miteinander verbunden sind.
3. Implantat nach einer der vorhergehenden Ausführungsformen, dadurch gekennzeichnet, dass jede Aussparung einen Zentralabschnitt (27a) sowie mindestens vier von diesem Zentralabschnitt kreuzförmig abgehende Seitenabschnitte (27b) aufweist.
4. Implantat nach einer der vorhergehenden Ausführungsformen, dadurch gekennzeichnet, dass die Breite b des Zentralabschnitts (27a) in Umfangsrichtung (U) mindestens 0,2 und maximal 5 Mal die Ausdehnung der Länge l des Zentralabschnitts beträgt.
5. Implantat nach einer der vorhergehenden Ausführungsformen, dadurch gekennzeichnet, dass die Länge jedes Seitenabschnitts (27b) gleich lang ist.
6. Implantat nach einer der vorhergehenden Ausführungsformen, dadurch gekennzeichnet, dass die Länge jedes Zentralabschnitts dieselbe Ausdehnung wie die Breite des Zentralabschnitts in Umfangsrichtung aufweist.
7. Implantat nach einer der vorhergehenden Ausführungsformen, dadurch gekennzeichnet, dass die Breite A des Knotens in Längsrichtung 0,2 bis 5 Mal die Breite B des Knotens in Umfangsrichtung U ist.
8. Implantat nach einer der vorhergehenden Ausführungsformen, dadurch gekennzeichnet, dass die Breite (B) des Knotens (24) in Umfangsrichtung U die Breite A des Knotens in Längsrichtung aufweist.
9. Implantat nach einer der vorhergehenden Ausführungsformen, dadurch gekennzeichnet, dass in die Aussparung (27) zwei einander gegenüberliegende Vorsprünge (28) hineinragen, die sich in Umfangsrichtung (U) erstrecken.
10. Implantat nach einer der vorhergehenden Ausführungsformen, dadurch gekennzeichnet, dass in die Aussparung zwei einander gegenüberliegende Vorsprünge (29) hineinragen, die sich in Längsrichtung (L) erstrecken.
11. Implantat nach einer der vorhergehenden Ausführungsformen, dadurch gekennzeichnet, dass die kreuzförmige durchgehende Aussparung (27) in jedem Knoten (24) eines Abschnitts der Grundstruktur (20) vorgesehen ist.
12. Implantat nach einer der vorhergehenden Ausführungsformen, dadurch gekennzeichnet, dass das Implantat als Herzklappenprothese ausgebildet ist und eine Grundstruktur (20) mit einer darin angeordnete Klappenanordnung umfasst.
13. Implantat nach Ausführungsform 12, dadurch gekennzeichnet, dass an der Grundstruktur (20) drei Klappensegel (30) und/oder ein Skirt (40) befestigt ist.
14. Implantat nach einer der vorhergehenden Ausführungsformen, dadurch gekennzeichnet, dass die Mehrzahl, bevorzugt alle, Knoten (24), die jeweils eine kreuzförmige durchgehende Aussparung (27) aufweisen, im Einströmbereich des Implantates angeordnet sind.

## Patentansprüche

1. Implantat (10) mit einer durchbrochenen, hohlzylinderförmigen und/oder hohlkegelstumpfförmigen Grundstruktur (20), die sich aus einer Vielzahl von Streben (21) und die Streben (21) miteinander verbindenden Knoten (24) zusammensetzt, wobei an jedem Knoten (24) jeweils mindestens zwei Streben (21) miteinander verbunden sind, **dadurch gekennzeichnet, dass** mindestens ein Teil der Knoten (24) jeweils eine kreuzförmige durchgehende Aussparung (27) aufweist.

2. Implantat nach Anspruch 1, **dadurch gekennzeichnet, dass** an einem Knoten (24) mindestens zwei Streben (21), vorzugsweise mindestens vier Streben (21) miteinander verbunden sind.

3. Implantat nach einem der vorhergehenden Ansprüche, **dadurch gekennzeichnet, dass** jede Aussparung einen Zentralabschnitt (27a) sowie mindestens vier von diesem Zentralabschnitt kreuzförmig abgehende Seitenabschnitte (27b) aufweist.

4. Implantat nach einem der vorhergehenden Ansprüche, **dadurch gekennzeichnet, dass** die Breite b des Zentralabschnitts (27a) in Umfangsrichtung (U) mindestens 0,2 und maximal 5 Mal die Ausdehnung der Länge l des Zentralabschnitts beträgt.

5. Implantat nach einem der vorhergehenden Ansprüche, **dadurch gekennzeichnet, dass** die Länge jedes Seitenabschnitts (27b) gleich lang ist.

6. Implantat nach einem der vorhergehenden Ansprüche, **dadurch gekennzeichnet, dass** die Länge jedes Zentralabschnitts dieselbe Ausdehnung wie die Breite des Zentralabschnitts in Umfangsrichtung aufweist.

7. Implantat nach einem der vorhergehenden Ansprüche, **dadurch gekennzeichnet, dass** die Breite A des Knotens in Längsrichtung 0,2 bis 5 Mal die Breite B des Knotens in Umfangsrichtung U ist.

8. Implantat nach einem der vorhergehenden Ansprüche, **dadurch gekennzeichnet, dass** die Breite (B) des Knotens (24) in Umfangsrichtung U die Breite A des Knotens in Längsrichtung aufweist.

9. Implantat nach einem der vorhergehenden Ansprüche, **dadurch gekennzeichnet, dass** in die Aussparung (27) zwei einander gegenüberliegende Vorsprünge (28) hineinragen, die sich in Umfangsrichtung (U) erstrecken.

10. Implantat nach einem der vorhergehenden Ansprüche, **dadurch gekennzeichnet, dass** in die Aussparung zwei einander gegenüberliegende Vorsprünge (29) hineinragen, die sich in Längsrichtung (L) erstrecken.

11. Implantat nach einem der vorhergehenden Ansprüche, **dadurch gekennzeichnet, dass** die kreuzförmige durchgehende Aussparung (27) in jedem Knoten (24) eines Abschnitts der Grundstruktur (20) vorgesehen ist.

12. Implantat nach einem der vorhergehenden Ansprüche, **dadurch gekennzeichnet, dass** das Implantat als Herzklappenprothese ausgebildet ist und eine Grundstruktur (20) mit einer darin angeordnete Klappenanordnung umfasst.

13. Implantat nach Anspruch 12, **dadurch gekennzeichnet, dass** an der Grundstruktur (20) drei Klappensegel (30) und/oder ein Skirt (40) befestigt ist.

14. Implantat nach einem der vorangehenden Ansprüche, **dadurch gekennzeichnet, dass** die Mehrzahl, bevorzugt alle, Knoten (24), die jeweils eine kreuzförmige durchgehende Aussparung (27) aufweisen, im Einströmbereich des Implantates angeordnet sind.
